# EUROPÄISCHE PATENTSCHRIFT

(11) **EP 0 636 012 B1**
(45) Veröffentlichungstag und Bekanntmachung des Hinweises auf die Patenterteilung: **08.07.1998**
(21) Anmeldenummer: 94908325.7
(22) Anmeldetag: 16.02.1994
(51) Int. Cl.: A61B 17/60

(54) **VORRICHTUNG ZUR VERLÄNGERUNG VON KNOCHEN**
BONE-EXTENDING DEVICE
DISPOSITIF D'EXTENSION DES OS

(30) Priorität: 18.02.1993 DE 4305047
(43) Veröffentlichungstag der Anmeldung: 01.02.1995
(73) Patentinhaber: ENDOCARE AG, CH-6343 Rotkreuz (CH)
(72) Erfinder: LANG, Karsten, D-1195 Berlin (DE); ZIPPEL, Hartmut, D-1040 Berlin (DE)
(74) Vertreter: Popp, Eugen, Dr.
(86) Internationale Anmeldenummer: EP9400445
(87) Internationale Veröffentlichungsnummer: WO9418898

(56) Entgegenhaltungen:
- EP-A- 0 194 187
- WO-A-88/05288
- DE-A- 3 345 276
- DE-A- 3 802 743
- FR-A- 2 129 735
- GB-A- 2 114 891

## Beschreibung

Die Erfindung betrifft eine Vorrichtung zur Verlängerung von Knochen gemäß dem Oberbegriff des Anspruchs 1.

Derartige Vorrichtungen, die neben einer Verlängerung von Knochen noch zusätzlich einem Knochenfragmenttransport innerhalb eines Extremitätenabschnittes, einer Wiederherstellung der Beweglichkeit von Gelenken, einer Korrektur bei angeborenen oder erworbenen Achsenfehlstellungen der Extremitäten, einer Behandlung frischer oder verzögert heilender Knochenbrüche, einer Ausheilung von Falschgelenken sowie einer Gelenkversteifung auf der Basis eines äußeren Festhalters, d.h. eines sog. Fixateur extern, dienen, sind hinlänglich bekannt. Diese Vorrichtungen zur Verlängerung von Knochen umfassen jeweils mindestens zwei Voneinander beabstandete, den Knochen wenigstens teilweise umgebende Rahmenelemente zur Aufnahme und Befestigung von Befestigungselementen sowie Spannelementen oder dgl. Die Befestigungselemente bzw. sogenannten Kraftabträger in Form von Spickdrähten, Kirschnerdrähten, Schrauben, Schanz'schen Schrauben etc. durchgreifen den Knochen etwa senkrecht zu dessen Längsrichtung bzw. werden unter aseptischen Sterilbedingungen in und/oder durch den Knochen eingebohrt und an den äußeren Rahmenelementen fixiert. Die Spannelemente oder dgl., die teleskopartig verlängerbar sind und die Rahmenelemente miteinander verbinden sowie relativ zueinander festlegen, sind zur Erzeugung von kontrollierbaren und kontinuierlichen Zug- und Druckkräften zur Verlängerung von Knochen, Knochenbruchbehandlung, Gelenkquengelung oder Gelenkversteifung vorgesehen.

Sämtliche dieser Vorrichtungen weisen den Nachteil einer ausgesprochen aufwendigen Konstruktion auf. So sind die Rahmenelemente bei diesen Vorrichtungen im allgemeinen als geschlossene Ringe ausgebildet, die mit einer einzigen Reihe von Bohrungen zur Aufnahme und Fixierung einerseits der Befestigungselemente und andererseits der Spannelemente oder dgl. versehen sind. Abgesehen von der Tatsache, daß derartige geschlossene Ringe nicht bei jedem Behandlungfall notwendig sind, verkomplizieren die geschlossenen Ringe die Handhabung der gesamten Vorrichtung, und zwar vor allem bei der Montage bzw. Demontage während des operativen Eingriffs. Zudem verhindern derartige geschlossene Ringe den unmittelbaren Zugang zu dem jeweiligen Knochen. Des weiteren ist die Anordnung der Befestigungselemente und Spannelemente oder dgl. an den jeweiligen Rahmenelementen infolge der einen Reihe von Bohrungen starr vorgegeben und insoweit nicht beliebig variierbar. So sind auch die Spannelemente oder dgl. bei diesen Vorrichtungen vielfach durch eine Gewindehülse mit zwei endseitig angeordneten Gewindestangen, die in die Gewindehülse einschraubbar sind, gebildet. Durch Verdrehen der Gewindehülse werden vielfach beide an dem jeweiligen Rahmenelement befestigten Gewindestangen gleichzeitig verlängert bzw. verkürzt. Auf diese Weise ist eine Feineinstellung des Abstandes zwischen den jeweiligen Rahmenelementen nur schwer möglich. Darüber hinaus weist die Gewindehülse bei diesen Vorrichtungen eine feste, unveränderbare Länge auf, so daß ein Spannelement oder dgl., das für einen verhältnismäßig kurzen Abstand zwischen den entsprechenden Rahmenelementen vorgesehen ist, für einen größeren Abstand zwischen den entsprechenden Rahmenelementen nicht verwendet werden kann. Ein vielseitiger Einsatz solcher Spannelemente oder dgl. und somit dieser Vorrichtungen ist daher ohne gegenseitigen Austausch der Spannelemente oder dgl. bei unterschiedlich großen Abständen zwischen den entsprechenden Rahmenelementen unmöglich.

Nicht zuletzt hieraus resultierend ist bei diesen Vorrichtungen weiterhin deren Handhabung sowohl während der Operationsphase als auch während der nachfolgenden Heilungsphase verhältnismäßig schwierig.

Schließlich hat sich in der Praxis noch zusätzlich herausgestellt, daß diese Vorrichtungen zumeist ein hohes Gewicht aufweisen, wodurch die Akzeptanz der Patienten stark beeinflußt wird.

Aus der FR-A-2 129 735 ist eine Vorrichtung zur Verlängerung von Knochen bekannt. Diese Vorrichtung umfaßt zwei voneinander beabstandete Rahmenelemente zur Aufnahme und Befestigung von Befestigungselementen. Diese Befestigungselemente greifen durch den Knochen etwa senkrecht zu dessen Längsrichtung. Die Vorrichtung hat weiterhin teleskopartig verlängerbare und die Rahmenelemente miteinander verbindende und relativ zueinander festlegende Spannelemente. Die Rahmenelemente sind U-förmig ausgebildet und weisen eine Reihe von Längsschlitzen zur Fixierung der Befestigungselemente und Spannelemente auf.

Durch diese U-förmige Ausgestaltung der Rahmenelemente ist die Vorrichtung zur Verlängerung von Knochen, zur medizinischen Heilbehandlung usw., die von Orthopäden, Traumatologen und Wiederherstellungschirurgen an allen Abschnitten der oberen und unteren Extremitäten eingesetzt werden kann, hinsichtlich ihrer Konstruktion und ihrer Handhabung sowohl während der Operationsphase als auch während der Heilungsphase besonders einfach. Durch diese U-förmige Ausgestaltung der Rahmenelemente ist nämlich ein weitestgehend ungestörter Zugang zum Knochen bzw. zum Operationsgebiet möglich. Die Folge hiervon ist eine enorme Erleichterung für den Operateur während der Operationsphase. Es lassen sich auch Korrekturmaßnahmen während der Heilungsphase von dem Operateur vornehmen.

Die Vorrichtung gemäß der FR-A-2 129 735 hat jedoch den Nachteil, daß die Anordnungsmöglichkeiten der Befestigungselemente und Spannelemente eingeschränkt sind.

Die Anordnungsmöglichkeiten sind weiterhin dadurch eingeschränkt, daß zwischen zwei benachbarten Schlitzen ein Zwischenraum erforderlich ist, in dem keine Spannelemente bzw. Befestigungselemente fixiert werden können.

Ausgehend vom Stand der Technik gemäß der FR-A 2 129 735 liegt der Erfindung die Augabe zugrunde, eine entsprechende Vorrichtung bereitzustellen, bei der die Vielfalt der Anordnungsmöglichkeiten der Befestigungselemente und der Spannelemente an den Rahmenelementen erhöht wird, ohne die Stabilität der Rahmenelemente zu beeinträchtigen.

Diese Aufgabe wird durch die kennzeichnenden Merkmale des Anspruches 1 gelöst, wonach die Längsschlitze in zwei oder mehreren Reihen angeordnet sind. Dies erhöht die Vielfalt der Anordnungsmöglichkeiten zweier oder mehrerer Befestigungselemente und/oder Spannelemente zueinander.

Mit einer Verwendung von Rahmenelementen unterschiedlicher Größe, die an den jeweiligen Durchmesser bzw. Umfang des Knochens oder der Extremität des Patienten angepaßt ist, läßt sich eine individuelle Anpassung und ein optimaler Tragekomfort erreichen.

Die Vorrichtung zur Verlängerung von Knochen nach der Erfindung läßt sich auf diese Weise vielseitig und/oder an die jeweiligen individuellen Gegebenheiten angepaßt verwenden, und zwar zur Fixierung der Befestigungselemente in Form von Drähten, Schrauben oder dgl. am jeweiligen Rahmenelement einerseits und der Spannelemente etc. der Rahmenelemente untereinander andererseits. Hierdurch können zusätzlich Rotationskorrekturen während der gesamten Behandlung vorgenommen werden. Schließlich können die erfindungsgemäßen Rahmenelemente auch ohne weiteres zu einem ovalen Vollring komplettiert werden, wodurch die Verwindungssteifigkeit der gesamten Vorrichtung zur Verlängerung von Knochen nach der Erfindung heraufgesetzt wird.

Nach den Merkmalen des Anspruchs 2 sind die Längsschlitze zweier jeweils benachbarter Reihen von Längsschlitzen einander überlappend angeordnet. Auf diese Weise ist das Rahmenelement nach der Erfindung mit wenigstens einem quasi durchgehenden Schlitz versehen, so daß die Befestigungselemente und Spannelemente beliebig an das jeweilige Rahmenelement in Abhängigkeit der individuellen Gegebenheiten des Knochens bzw. der Extremität, d.h. beispielsweise auch des Verlaufes der Nerven, Arterien und Venen etc., angeordnet werden können. Auch läßt sich somit der Abstand zu den zwei benachbarten Befestigungselementen und/oder Spannelementen auf ein Minimum reduzieren.

Weiterhin liegt es im Rahmen der Erfindung nach den Ansprüchen 3 bis 5, die Reihen von Längsschlitzen durch wenigstens einen Querschlitz zu unterbrechen, mindestens einen Längsschlitz einer Reihe von Längsschlitzen hin zu einer benachbarten Reihe von Längsschlitzen verlaufend auszubilden und wenigstens einen Längsschlitz einer Reihe von Längsschlitzen am Ende des halbovalen Rahmenelements stirnseitig frei zugängig zu machen. Auf diese Weise wird die Handhabbarkeit des halbovalen Rahmenelements und damit zusammenhängend der Vorrichtung zur Verlängerung von Knochen etc. nach der Erfindung insgesamt verbessert und dessen bzw. deren vielseitige Verwendungsmöglichkeit zusätzlich erhöht.

Von besonderem Interesse für eine weitere Vereinfachung der Handhabung der erfindungsgemäßen Vorrichtung zur Verlängerung von Knochen etc. sind darüber hinaus die Merkmale des Anspruchs 6, und zwar auch unabhängig von den Ansprüchen 1 bis 5, daß nämlich an den Rahmenelementen festlegbare Klemmschrauben zur Aufnahme und Fixierung von den Befestigungselementen vorgesehen sind. Dabei sind die Befestigungselemente als sog. Kraftabträger in Form von Spickdrähten, Kirschnerdrähten oder dgl. ausgebildet, die am Knochen bzw. an der Extremität unter Beobachtung des anatomischen Nerven- und Gefäßverlaufes in eingebrachte Bohrungen gekreuzt befestigt werden.

Vorteilhafterweise sind die Klemmschrauben entsprechend Anspruch 7 jeweils derart ausgebildet, daß die Klemmschrauben für die Spickdrähte auf dem Rahmenelement großflächig und damit rotationsstabil zur Anlage gelangen. Infolge einer solchen großflächigen Anlage wird eine erhöhte Reibungskraft erzeugt, die zwischen der jeweiligen Klemmschraube und dem entsprechenden Rahmenelement wirkt. Ein Kontern der jeweiligen Klemmschraube während des Anziehens wird daher überflüssig.

Weiterhin liegt es gemäß Anspruch 8 im Rahmen der Erfindung, die Klemmschrauben zweigeteilt auszubilden. Dabei umfaßt das eine Teil einen Schraubenkopf und ein Schraubengewinde, zwischen denen ein Vorsprung mit einer Bohrung, vorzugsweise einer Mittelbohrung, zur Aufnahme des Spickdrahtes angeordnet ist. Der Vorsprung weist einen Durchmesser auf, der kleiner ist als der Durchmesser des Schraubenkopfes, jedoch größer als der Durchmesser des Schraubengewindes. Dabei stellt weiterhin das andere Teil eine Klemmscheibe oder dgl. dar, die mit einer dem Schraubenkopf zugewandten, dem Spickdraht der beaufschlagenden Seite und mit einer dem Schraubenkopf abgewandten, flächig auf dem Rahmenelement aufliegenden Seite versehen ist. Die eine Seite der Klemmscheibe oder dgl. beaufschlagt und verankert somit den von der Bohrung aufgenommenen, unmittelbar unterhalb des Schraubenkopfes liegenden Spickdraht, während die andere Seite mit dem Rahmenelement großflächig in Berührung steht.

Die Merkmale nach Anspruch 9, daß die Klemmscheibe oder dgl. eine zu dem Vorsprung korrespondierende und diesen aufnehmende Ausnehmung aufweist, sorgt zusätzlich für eine stabile und dauerhafte Verankerung des jeweiligen Spickdrahtes in der Klemmschraube und somit an dem Rahmenelement.

Insbesondere ist die Klemmscheibe oder dgl. nach den Maßnahmen des Anspruchs 10 an ihrer dem Schraubenkopf zugewandten Seite mit wenigstens zwei einander diametral gegenüberliegenden, radial verlaufenden Kerben, Nuten oder dgl. versehen, die den Spickdraht, welcher den Vorsprung der Klemmschraube durchgreift, zumindest teilweise aufnehmen. Hierdurch wird eine Verbiegung oder gar ein Bruch des jeweiligen Spickdrahtes selbst bei einer zu großen Anzugskraft durch die die Klemmschraube konternden Muttern verhindert.

In vorteilhafter Weise sind die Klemmschrauben nach den Merkmalen des Anspruchs 11 jeweils mit einer Mutter konterbar, die eine Scheibe oder dgl. umfaßt, welche am Rahmenelement zur Anlage kommt und geringfügig verkippbar ist. Eine solche konstruktive Ausgestaltung der Muttern vereinfacht die Handhabung der Vorrichtung zur Verlängerung von Knochen insgesamt, vor allem während der Operation, und ermöglicht zudem eine hohe mechanische Stabilität der gesamten Schraubenverbindung.

Von großer Bedeutung für eine zusätzliche Vereinfachung der Handhabbarkeit der Vorrichtung zur Verlängerung von Knochen etc. nach der Erfindung sind weiterhin die Merkmale nach Anspruch 12, und zwar auch unabhängig von den Maßnahmen nach den Ansprüchen 1 bis 11. Demnach umfassen die teleskopartig verlängerbaren, die Rahmenelemente miteinander verbindenden und relativ zueinander festlegenden Spannelemente oder dgl. wenigstens zwei, vorzugsweise drei Gewindeabschnitte, die wenigstens teilweise ineinander greifen. Neben einer besonders genauen Feineinstellung lassen sich sowohl kleine als auch große Abstände zwischen einzelnen Rahmenelementen durch ein und dieselben Spannelemente oder dgl. entsprechend dem jeweiligen operativen Erfordernis erhalten, ohne einzelne Spannelemente während der Operationsphase, insbesondere aber während der Behandlungs- und Heilungsphase, gegen Spannelemente anderer Länge austauschen zu müssen. Infolge einer solchen Längenverstellbarkeit der Spannelemente oder dgl. selbst über eine große Wegstrecke ist ein vielseitiger Einsatz dieser Spannelemente und somit der gesamten Vorrichtung zur Verlängerung von Knochen etc. nach der Erfindung sichergestellt.

Nach einem weiteren Merkmal entsprechend Anspruch 13 ist ein erster Gewindeabschnitt von einer zentralen Gewindespindel und einer zentralen, mit der Gewindespindel zusammenwirkenden Gewindehülse gebildet, die jeweils endseitig, voneinander abgewandt einen zweiten und dritten Gewindeabschnitt einschraubbar aufnehmen.

In weiterer Ausgestaltung der Erfindung ist gemäß Anspruch 14 vorgesehen, den ersten Gewindeabschnitt zu dem zweiten und dritten Gewindeabschnitt entgegengesetzt drehend auszubilden. Insbesondere entsprechend Anspruch 15 sind der erste Gewindeabschnitt linksdrehend und der zweite sowie dritte Gewindeabschnitt rechtsdrehend ausgebildet. Auf diese Weise lassen sich die Spannelemente oder dgl. gegenüber der Länge des ersten Gewindeabschnitts um wenigstens die doppelte Länge ausfahren, so daß während der Behandlung kein Wechsel des jeweiligen Spannelementes oder dgl. erfolgen muß.

Durch die Maßnahmen nach den Ansprüchen 16 und 17, den ersten Gewindeabschnitt mit einer Markierung auszustatten, insbesondere die zentrale Gewindespindel in Mittellängsrichtung mit einer Nut, Kerbe oder dgl. sowie die Gewindehülse im Bereich ihrer Öffnung mit einer umfangsmäßig angeordneten Skalierung oder dgl. zu versehen, läßt sich eine genau vorherbestimmbare, exakt dosierte Verlängerung des jeweiligen Spannelementes erreichen.

In vorteilhafter Weise sind der zweite und dritte Gewindeabschnitt gemäß Anspruch 18 jeweils als Gewindestange ausgebildet.

Im weiteren Rahmen der Erfindung ist nach Anspruch 19 vorgesehen, daß die beiden Enden der Gewindestange koaxial zu der Längsachse der Gewindestange verlaufen. Die gegenseitige Verbindung und Festlegung der Rahmenelemente kann in diesem Zusammenhang mittels jeweils verschieden langer Gewindestangen realisiert sein.

In alternativer Ausgestaltung der Erfindung entsprechend Anspruch 20 können die beiden Enden der Gewindestange ebenso zueinander parallel verlaufen, wodurch Rahmenteile unterschiedlicher Größe bzw. auch unterschiedlichen Durchmessers mühelos miteinander verbunden oder zusammengefügt werden können. Dabei umfaßt die Gewindestange nach Merkmal des Anspruchs 21 einen mittleren Bereich, der gegenüber den beiden Enden der Gewindestange bis zu einem Winkel von 90° abgewinkelt ausgebildet ist.

In weiterer alternativer Ausgestaltung der Erfindung entsprechend Anspruch 22 können die beiden Enden der Gewindestange auch zueinander bis zu einem Winkel von 180° abwinkelbar sein. Dabei umfaßt die Gewindestange gemäß Maßnahmen nach Anspruch 23 einen mittleren Bereich, in dem ein scharnierartiges Gelenk oder dgl. angeordnet ist. Eine derartige Gewindestange findet hauptsächlich bei gelenküberbrückenden Vorrichtungen zur Verlängerung von Knochen oder Extremitäten bzw. bei beabsichtigten Achsenkorrekturen Verwendung. Dabei gestattet eine solche Gewindestange mit einem scharnierartigen Gelenk aufgrund ihrer variablen Positionierungsmöglichkeit Korrekturen in allen Freiheitsgraden.

Die Verankerung jeder geraden Gewindestange, gebogenen Gewindestange oder Gewindestange mit scharnierartigem Gelenk an den entsprechenden Rahmenelementen kann endseitig jeweils über eine Mutter erfolgen, die eine am Rahmenelement zur Anlage kommende, geringfügig verkippbare Scheibe oder dgl. zur Vereinfachung der Handhabung der erfindungsgemäßen Vorrichtung sowie zur Erhöhung der mechanischen Stabilität umfaßt.

Darüber hinaus sind der zweite und dritte Gewindeabschnitt entsprechend den Maßnahmen nach Anspruch 24 auch schräg zu dem jeweiligen Rahmenelement verlaufend an dem Rahmenelement befestigbar. Auf diese Weise können Achsenabweichungen der Knochen oder Extremitäten ausgeglichen werden. Als besonders vorteilhaft hat es sich in diesem Zusammenhang nach Anspruch 25 erwiesen, den zweiten und dritten Gewindeabschnitt über eine Mutter mit dem Rahmenelement zugewandter Kalottenform und eine Zwischenscheibe mit zur Kalottenform der Mutter entsprechender, dem Rahmenelement abgewandter kalottenförmiger Auflage an dem jeweiligen Rahmenelement zu befestigen. Hierdurch ist eine endseitige Fixierung von zweitem und dritten Gewindeabschnitt bis zu einem abweichenden Winkel von etwa 45°, insbesondere von ca. 30°, gegenüber der Horizontalebene möglich.

Schließlich bestehen die Rahmenelemente und/oder die Spannelemente und/oder die Klemmschrauben entsprechend der Maßnahme nach Anspruch 26 aus Aluminium, insbesondere aus Duraluminium. Die Folge hiervon ist eine Vorrichtung zur Verlängerung von Knochen oder Extremitäten mit ausgesprochen geringem Gewicht und einer damit einhergehenden hohen patientenseitigen Akzeptanz.

Weitere Merkmale, Vorteile und Einzelheiten der Erfindung ergeben sich aus der nachfolgenden Beschreibung einiger bevorzugter Ausführungsformen der Erfindung sowie anhand der Zeichnung. Hierbei zeigen:
- Fig. 1: eine perspektivische Seitenansicht einer Ausführungsform einer erfindungsgemäß ausgebildeten Vorrichtung zur Verlängerung von Knochen;
- Fig. 2: eine Draufsicht auf zwei Ausführungsformen von erfindungsgemäß ausgebildeten halbovalen Rahmenelementen in vergrößertem Maßstab;
- Fig. 3: eine Draufsicht auf ein durch die beiden halbovalen Rahmenelemente gemäß Fig. 2 gebildetes Oval;
- Fig. 4: eine perspektivische Seitenansicht einer Ausführungsform einer erfindungsgemäß ausgebildeten Klemmschraube mit einer erfindungsgemäßen Klemmscheibe sowie Mutter;
- Fig. 5: eine Seitenansicht der erfindungsgemäßen Klemmschraube nach Fig. 4 in verkleinertem Maßstab;
- Fig. 6: eine perspektivische Draufsicht auf die erfindungsgemäße Klemmscheibe nach Fig. 4 entsprechend Pfeil VI in verkleinertem Maßstab;
- Fig. 7: eine perspektivische Unteransicht auf die erfindungsgemäße Mutter nach Fig. 4 entsprechend Pfeil VII in verkleinertem Maßstab;
- Fig. 8: eine Seitenansicht einer Ausführungsform eines erfindungsgemäß ausgebildeten Spannelementes oder dgl. in vergrößertem Maßstab;
- Fig. 9A, 9B, 9C, 9D: schematische Draufsichten auf Ausführungsformen eines erfindungsgemäß ausgebildeten zweiten und/oder dritten Gewindeabschnittes;
- Fig. 10A und 10B: schematische Draufsichten auf andere Ausführungsformen eines erfindungsgemäß ausgebildeten zweiten und/oder dritten Gewindeabschnittes;
- Fig. 10C: eine perspektivische Ansicht auf eine andere Ausführungsform eines erfindungsgemäß ausgebildeten scharnierartigen Gelenks;
- Fig. 11: eine schematische Seitenansicht auf eine Ausführungsform der erfindungsgemäßen Vorrichtung zusammen mit einem Patientenbein zur Veranschaulichung der Wirkungsweise von zweitem und/oder dritten Gewindeabschnitt entsprechend Fig. 10A und 10B in verkleinertem Maßstab;
- Fig. 12: eine teilweise abgebrochene Schnittansicht einer Ausführungsform einer erfindungsgemäß kalottenartig ausgebildeten Mutter mit Zwischenscheibe zur schrägen Fixierung eines erfindungsgemäßen Spannelementes.

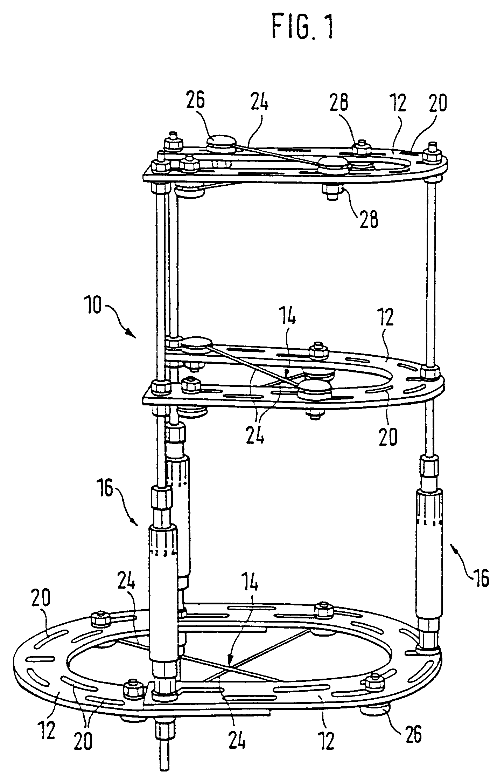

Die in Fig. 1 dargestellte Ausführungsform einer Vorrichtung 10 zur Verlängerung von Knochen oder Extremitäten etc. umfaßt insgesamt vier Rahmenelemente 12, die voneinander beabstandet sind und den jeweiligen Knochen (nicht dargestellt) wenigstens teilweise entsprechend den beiden oberen Rahmenelementen 12 bzw. vollständig entsprechend den beiden unteren Rahmenelementen 12 umgeben. Die Rahmenelemente 12 sind zur Aufnahme und Befestigung einerseits von Befestigungselementen 14 und andererseits von Spannelementen 16 oder dgl. vorgesehen.

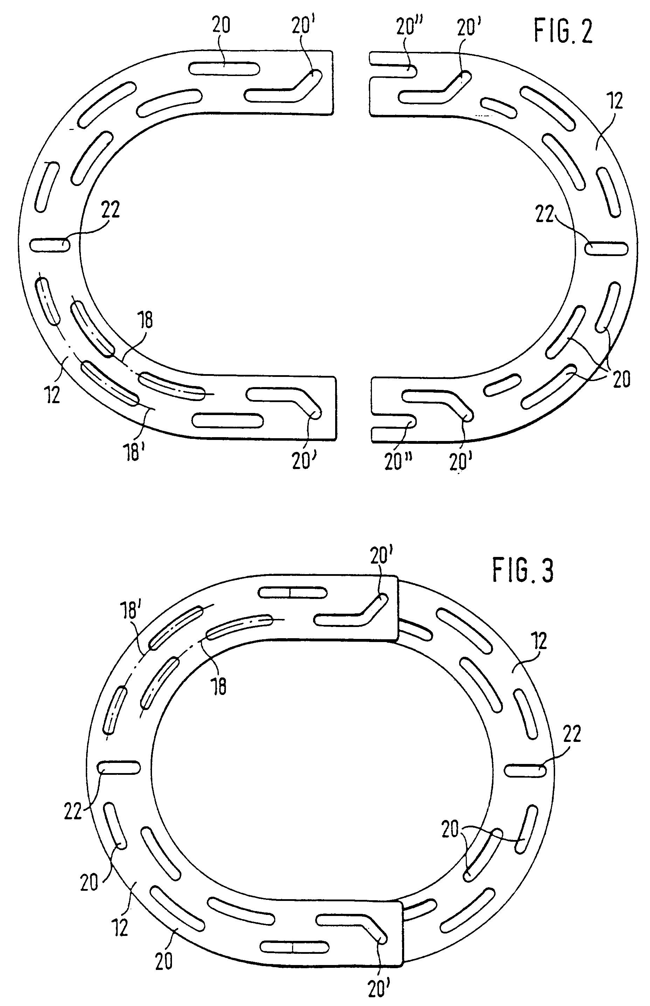

Gemäß den Figuren 1 bis 3 sind die Rahmenelemente 12 jeweils U-förmig, halboval oder dgl. ausgebildet. Auf diese Weise wird während der Operation ein nahezu ungestörter Zugang zum Operationsgebiet und damit eine Erleichterung der Handhabung für den Operateur während des Eingriffs erreicht. Darüber hinaus können jeweils zwei U-förmig, halboval oder dgl. ausgebildete Rahmenelemente 12 gemäß Figuren 1 und 3 ohne Schwierigkeit schon während des operativen Eingriffs oder nachträglich bei der eigentlichen Behandlung zu einem ovalen Vollring, Kreisring etc. zusammengesetzt werden, wodurch gleichzeitig die Verbindungssteifigkeit der gesamten Vorrichtung 10 zur Verlängerung von Knochen oder Extremitäten erhöht wird.

Die Rahmenelemente 12 weisen darüber hinaus vorzugsweise zwei - wie in den Figuren 1 bis 3 dargestellt - oder mehrere Reihen 18, 18' von Längsschlitzen 20, 20' 20'' auf. Die Längsschlitze 20, 20', 20'' dienen der Fixierung der Befestigungselemente 14 und Spannelemente 16 an den Rahmenelementen 12. Die Längsschlitze 20, 20', 20'' der beiden benachbarten Reihen 18, 18' von Längsschlitzen 20, 20', 20'' sind dabei einander überlappend angeordnet. Die Überlappung entspricht gemäß den Figuren 2 und 3 in etwa der Breite eines jeden Längsschlitzes 20, 20', 20''. Infolge dieser sich einander überlappenden Längsschlitze 20, 20', 20'' ist eine verbesserte Flexibilität bezüglich der Einsatzmöglichkeiten sowie Handhabung der Vorrichtung 10 zur Verlängerung von Knochen ermöglicht.

Weiterhin sind bei den Rahmenelementen 12 nach den Figuren 2 und 3 die Reihen 18, 18' von Längsschlitzen 20, 20', 20'' durch wenigstens einen Querschlitz 22 unterbrochen. Zudem ist wenigstens ein Längsschlitz 20' der Reihe 18 von Längsschlitzen 20, 20' hin zu der benachbarten Reihe 18' von Längsschlitzen 20, 20'' verlaufend ausgebildet. Schließlich ist wenigstens ein Längsschlitz 20'' der Reihe 18' von Längsschlitzen 20, 20'' am Ende des U-förmigen, halbovalen etc. Rahmenelements 12 entsprechend einer Ausnehmung stirnseitig frei zugänglich. Durch eine solche Ausbildung der Längsschlitze 20, 20', 20'' an dem Rahmenelement 12 ist eine zusätzliche Variationsmöglichkeit bei der Montage von Befestigungselementen 14 und Spannelementen 16 an dem jeweiligen Rahmenelement 12 gegeben.

Wie aus Fig. 1 deutlich hervorgeht, handelt es sich bei den Befestigungselementen 14 um sog. Kraftabträger in Form von Spickdrähten 24, Kirschnerdrähten, Schrauben, Schanz'schen Schrauben oder dgl., die unter aseptischen Sterilbedingungen in bzw. durch den Knochen oder die Extremität ein- bzw. durchgebohrt werden, d.h. den Knochen etwa senkrecht zu dessen Längsrichtung durchgreifen und anschließend an dem jeweiligen Rahmenelement 12 fixiert werden.

Zur Aufnahme und Befestigung wiederum von den Befestigungselementen 14 in Form der Spickdrähte 24 sind an den Rahmenelementen 12 festlegbare Klemmschrauben 26 vorgesehen. Die Klemmschrauben 26 sind dabei jeweils derart ausgebildet, daß die Klemmschrauben 26 für die Spickdrähte 24 auf dem Rahmenelement 12 großflächig zur Anlage kommen. Auf diese Weise sind die Klemmschrauben 26 rotationsstabil, so daß beim Kontern mittels einer Mutter lediglich ein Schraubenschlüssel, Maulschlüssel, Ringschlüssel usw. für eben ausschließlich die Mutter 28 notwendig ist.

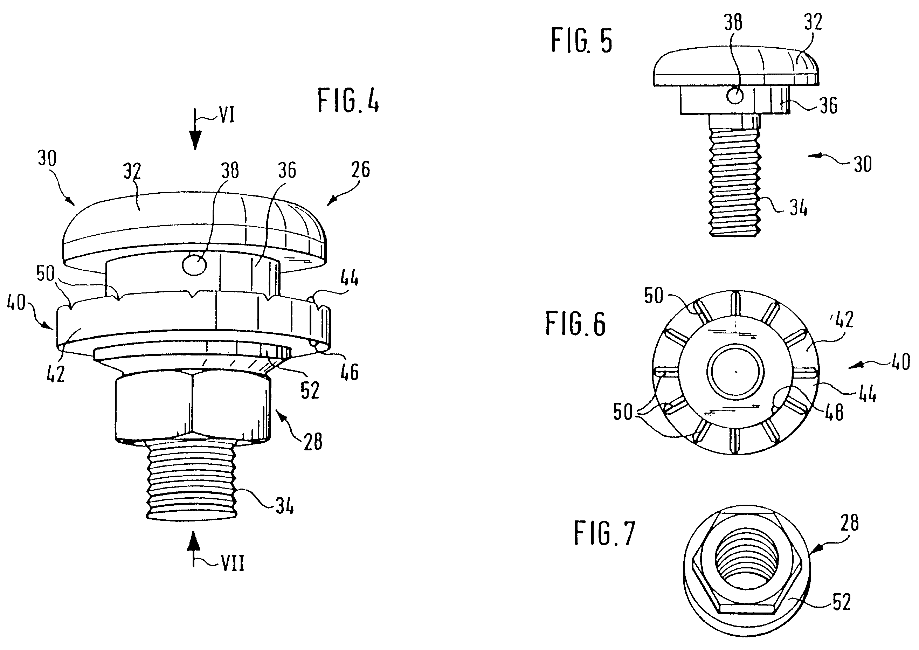

Die Klemmschrauben 26 sind gemäß den Figuren 4 bis 6 zweigeteilt ausgebildet.

Das eine Teil 30 umfaßt einen Schraubenkopf 32 und ein Schraubengewinde 34. Zwischen Schraubenkopf 32 und Schraubengewinde 34 ist weiterhin ein Vorsprung 36 angeordnet, der mit einer Bohrung, vorzugsweise einer Mittelbohrung 38, zur Aufnahme des nicht dargestellten Spickdrahtes 24 versehen ist. Der Vorsprung 36 selbst besitzt einen Durchmesser, der kleiner ist als der Durchmesser des Schraubenkopfes 32 und größer ist als der Durchmesser des Schraubengewindes 34.

Das andere Teil 40 stellt eine Klemmscheibe 42 oder dgl. dar. Die Klemmscheibe 42 beaufschlagt den Spickdraht 24 mit einer dem Schraubenkopf 32 zugewandten Seite 44 und liegt flächig auf dem Rahmenelement 12 mit der anderen, dem Schraubenkopf 32 abgewandten Seite 46 auf. Die Klemmscheibe 42 oder dgl. besitzt eine zu dem Vorsprung 36 korrespondierende und den Vorsprung 36 aufnehmende Ausnehmung 48. Weiterhin ist die Klemmscheibe 42 oder dgl. an ihrer dem Schraubenkopf 32 zugewandten Seite 44 mit einer Vielzahl von einander diametral gegenüberliegenden, radial verlaufenden Kerben 50, Nuten oder dgl. versehen, die der teilweisen Aufnahme des den Vorsprung 36 über die Bohrung 38 durchgreifenden Spickdrahtes 24 dienen.

Entsprechend den Figuren 4 und 7 sind die Klemmschrauben 26 jeweils mit der Mutter 28 konterbar. Die Mutter 28 umfaßt eine Scheibe 52, Unterlegscheibe oder dgl., die am Rahmenelement 12 zur Anlage bringbar ist.

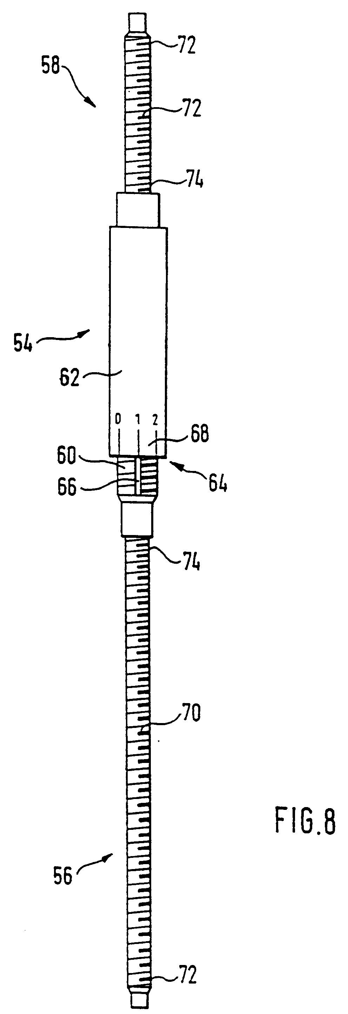

Die Spannelemente 16 oder dgl., wie in den Figuren 1 und 8 gezeigt, sind teleskopartig verlängerbar, verbinden die Rahmenelemente 12 jeweils miteinander und legen die Rahmenelemente 12 relativ zueinander fest. Dabei umfaßt jedes Spannelement 16 oder dgl. nach Fig. 8 mindestens zwei, vorzugsweise drei Gewindeabschnitte 54, 56, 58, die wenigstens teilweise ineinander greifen.

Der erste Gewindeabschnitt 54 ist von einer zentralen Gewindespindel 60 und einer zentralen Gewindehülse 62 gebildet, die mit der Gewindespindel 60 zusammenwirkt. Die Gewindespindel 60 wie auch die Gewindehülse 62 nehmen jeweils endseitig, voneinander abgewandt den zweiten und dritten Gewindeabschnitt 56, 58 einschraubbar auf.

In vorteilhafter Weise ist der erste Gewindeabschnitt 54 zu dem zweiten und dritten Gewindeabschnitt 56, 58 entgegengesetzt drehend ausgebildet. Insbesondere sind für den ersten Gewindeabschnitt 54 ein Linksgewinde und für den zweiten sowie dritten Gewindeabschnitt 56, 58 ein Rechtsgewinde vorgesehen.

Der erste Gewindeabschnitt 54 ist weiterhin mit einer Markierung 64 ausgestattet, um das jeweilige Spannelement 12 um eine genau definierbare Wegstrecke zu verlängern bzw. zu verkürzen. Zu diesem Zweck ist die zentrale Gewindespindel 60 in Mittellängsrichtung mit einer Nut 66, Kerbe oder dgl. versehen, während die Gewindehülse 62 im Bereich ihrer Öffnung eine umfangsmäßig angeordnete Skalierung 68 oder dgl. aufweist.

Wie aus Fig. 8 deutlich hervorgeht, sind der zweite und dritte Gewindeabschnitt 56, 58 jeweils als Gewindestange 70 ausgebildet. Die beiden Enden 72, 74 einer jeden Gewindestange 70 verlaufen dabei zu der Mittellängsachse der Gewindestange 70 koaxial.

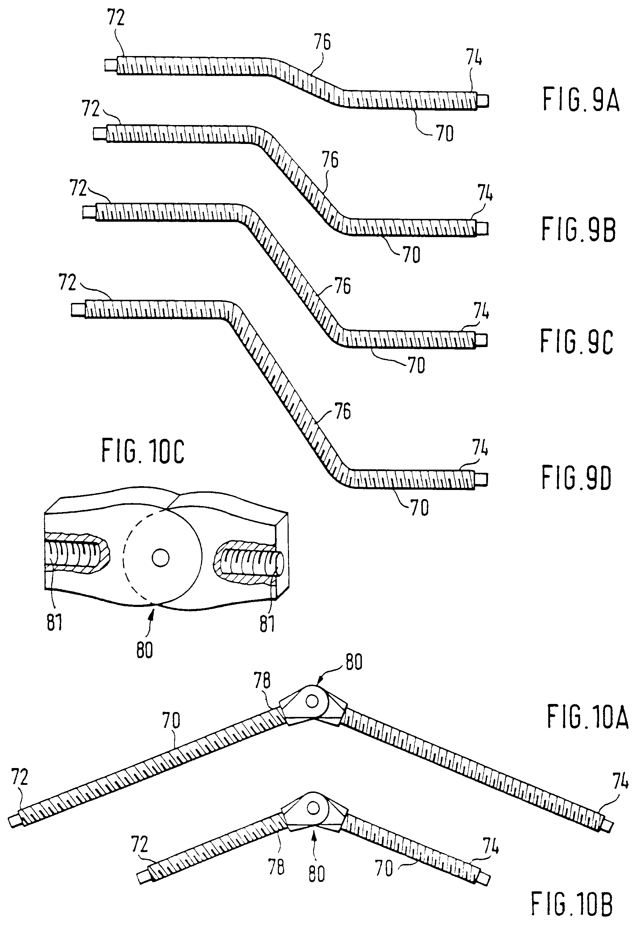

Entsprechend den Figuren 9A bis 9D können die Gewindestangen 70 in alternativer Ausgestaltung auch gebogen sein, wodurch selbst unterschiedliche große Rahmenelemente 12 ohne Schwierigkeit zusammensetzbar sind. Die beiden Enden 72, 74 der Gewindestange 70 verlaufen in diesem Fall zueinander parallel, wobei die Gewindestange 70 einen mittleren Bereich 76 umfaßt, der gegenüber den beiden Enden 72, 74 der Gewindestange bis zu einem Winkel von 90° abgewinkelt ausgebildet ist. Der mittlere Bereich 76 kann in diesem Zusammenhang auch eine unterschiedliche Länge besitzen.

In weiterer alternativer Ausgestaltung der Gewindestange 70 sind nach den Figuren 10A und 10B deren beiden Enden 72, 74 zueinander bis zu einem Winkel von 180° abwinkelbar. Zu diesem Zweck umfaßt die Gewindestange 70 einen mittleren Bereich 78, in dem ein scharnierartiges Gelenk 80 oder dgl. angeordnet ist. Eine solche Gewindestange 70 kommt vornehmlich bei gelenküberbrückenden Konstruktionen bzw. bei geplanten Achsenkorrekturen zum Einsatz. Entsprechend Fig. 10C weist das scharnierartige Gelenk 80 od. dgl. vorzugsweise zwei Gewindebohrungen 81 auf, in welche sich Gewindestangen 70 unterschiedlichster Bauart entsprechend der jeweiligen operativen Gegebenheiten, d.h. also gerade verlaufende Gewindestangen 70 oder auch gebogene Gewindestangen 70 gemäß den Fig. 9A bis 9D, einschrauben lassen. Die hieraus resultierende Vielseitigkeit erhöht zusätzlich das Einsatzspektrum der Vorrichtung 10 zur Verlängerung von Knochen oder Extremitäten.

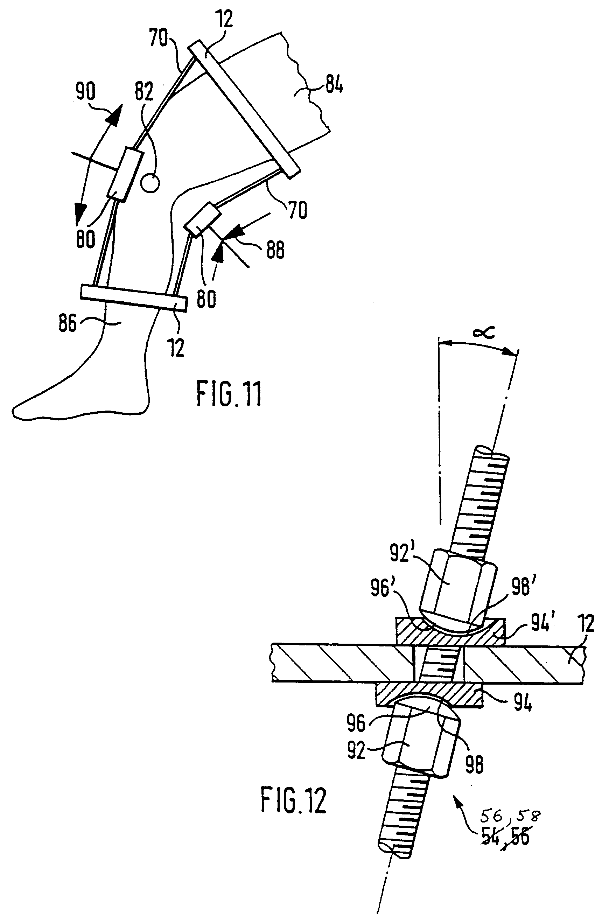

Entsprechend Fig. 11 läßt sich beispielsweise mittels Gewindestange 70 nach den Figuren 10A und 10B eine allmähliche Winkeleinstellung des hier gezeigten Kniegelenkes 82 zwischen Oberschenkel 84 und Unterschenkel 86 erreichen, indem der jeweilig von den beiden schematisch dargestellten Gewindestangen 70 eingeschlossene Winkel zwischen den beiden Rahmenelementen 12 vergrößert wird. Gleichzeitig ist es in der zuvor erläuterten Weise über Spannelemente 16 insbesondere gemäß Fig. 8 möglich, die Wegstrecke des hinteren bzw. unteren Spannelementes 16 mit den Gewindestangen 70 zwischen den beiden Rahmenelementen 12 zu verkürzen und die Wegstrecke des vorderen bzw. oberen Spannelementes 16 mit den Gewindestangen 70 zwischen den beiden Rahmenelementen 12 zu verlängern, und umgekehrt. Insoweit erfolgt eine Kraftaufbringung auf die beiden Rahmenelemente 12 im hinteren Bereich entsprechend Doppelpfeil 88 und im vorderen Bereich entsprechend Doppelpfeil 90.

Nach Fig. 12 sind der zweite und dritte Gewindeabschnitt 56, 58 ebenso schräg zu dem jeweiligen Rahmenelement 12 verlaufend an dem Rahmenelement 12 befestigbar. Dementsprechend können der zweite und dritte Gewindeabschnitt 56, 58 über eine Mutter 92 und eine Zwischenscheibe 94 an dem jeweiligen Rahmenelement 12 fixiert werden. Die Mutter 92 ist in dem dem Rahmenelement 12 zugewandten Bereich 96 kalottenartig ausgebildet. Demgegenüber weist die Zwischenscheibe 94 eine kalottenförmige Auflage 98 auf, die mit dem kalottenförmigen Bereich 96 der Mutter 92 korrespondiert, jedoch dem Rahmenelement 12 abgewandt ist. In entsprechender Weise werden der zweite und dritte Gewindeabschnitt 56, 58 von einer einen kalottenförmigen Bereich 96' aufweisenden Mutter 92' und einer Zwischenscheibe 94' mit einer kalottenförmigen Auflage 98' gekontert.

Die Rahmenelemente 12 und/oder die Spannelemente 16 und/oder die Klemmschrauben 26 bestehen zur Gewichtsverringerung der Vorrichtung 10 zur Verlängerung von Knochen oder Extremitäten insgesamt aus Aluminium und vorzugsweise aus Duraluminium, wodurch die Vorrichtung 10 eine zusätzliche Patientenakzeptanz erfährt.

Die vorliegende Erfindung ist nicht auf die vorangehenden Ausführungsbeispiele beschränkt. Vielmehr ist es durchaus auch möglich, anstelle von teleskopartig verlängerbaren Spannelementen 16 oder dgl. auch starre, d.h. nicht verlängerbare Gewindestangen oder dgl. zwischen die jeweiligen Rahmenelemente 12 einzufügen und an diesen zu befestigen. Ebenso ist es denkbar, zusätzlich zu den teleskopartig verlängerbaren Spannelementen 16 oder dgl. starre, nicht verlängerbare Gewindestangen zur kurzzeitigen Stabilitätsvergrößerung der gesamten Vorrichtung 10 nach der Erfindung zwischen den jeweiligen Rahmenelementen 12 vorzusehen.

## Patentansprüche

1. Vorrichtung zur Verlängerung von Knochen, umfassend mindestens zwei voneinander beabstandete, den Knochen wenigstens teilweise umgebende Rahmenelemente (12) zur Aufnahme und Befestigung von den Knochen etwa senkrecht zu dessen Längsrichtung durchgreifenden Befestigungselementen (14) und teleskopartig verlängerbaren und die Rahmenelemente miteinander verbindenden sowie relativ zueinander festlegenden Spannelementen (16), wobei die Rahmenelemente (12) jeweils U-förmig oder halboval ausgebildet sind und Längsschlitze (20, 20', 20'') zur Fixierung der Befestigungselemente (14) und Spannelemente (16) aufweisen,
**dadurch gekennzeichnet,**
daß die Längsschlitze (20, 20', 20'') in zwei oder mehreren Reihen angeordnet sind.

2. Vorrichtung nach Anspruch 1,
**dadurch gekennzeichnet**,
daß die Längsschlitze (20, 20', 20'') zweier jeweils benachbarter Reihen (18, 18') von Längsschlitzen (20, 20', 20'') einander überlappend angeordnet sind.

3. Vorrichtung nach Anspruch 1 und/oder 2,
**dadurch gekennzeichnet**,
daß die Reihen (18, 18') von Längsschlitzen (20, 20', 20'') durch wenigstens einen Querschlitz (22) unterbrochen sind.

4. Vorrichtung nach wenigstens einem der Ansprüche 1 bis 3,
**dadurch gekennzeichnet**,
daß wenigstens ein Längsschlitz (20') einer oder mehrerer der Reihen (18 bzw. 18') von Längsschlitzen (20, 20', 20'') hin zu einer benachbarten Reihe (18' bzw. 18) von Längsschlitzen (20, 20', 20'') verlaufend ausgebildet ist.

5. Vorrichtung nach wenigstens einem der Ansprüche 1 bis 4,
**dadurch gekennzeichnet**,
daß wenigstens ein Längsschlitz (20'') einer oder mehrerer der Reihen (18, 18') von Längsschlitzen (20, 20', 20'') am Ende des U-förmigen, halbovalen Rahmenelements (12) stirnseitig frei zugänglich ist.

6. Vorrichtung nach wenigstens einem der Ansprüche 1 bis 5,
**dadurch gekennzeichnet**,
daß an den Rahmenelementen (12) festlegbare Klemmschrauben (26) zur Aufnahme und Fixierung von den Befestigungselementen (14) vorgesehen sind, wobei die Befestigungselemente (14) als Spickdrähte (24) ausgebildet sind.

7. Vorrichtung nach Anspruch 6,
**dadurch gekennzeichnet**,
daß die Klemmschrauben (26) jeweils derart ausgebildet sind, daß die Klemmschrauben (26) für die Spickdrähte (24) auf dem Rahmenelement (12) großflächig und damit rotationsstabil zur Anlage kommen.

8. Vorrichtung nach Anspruch 6 oder 7,
**dadurch gekennzeichnet**,
daß die Klemmschrauben (26) zweigeteilt ausgebildet sind, wobei das eine Teil (30) einen Schraubenkopf (32) und ein Schraubengewinde (34) umfaßt, zwischen denen ein Vorsprung (36) mit einer Bohrung, vorzugsweise einer Mittelbohrung (38), zur Aufnahme des Spickdrahtes (24) mit einem Durchmesser angeordnet ist, der kleiner als der Durchmesser des Schraubenkopfes (32) und größer als der Durchmesser des Schraubengewindes (34) ist, und wobei das andere Teil (40) eine Klemmscheibe (42) mit einer dem Schraubenkopf (32) zugewandten, den Spickdraht beaufschlagenden Seite (44) und mit einer dem Schraubenkopf (32) abgewandten, flächig auf dem Rahmenelement (12) aufliegenden Seite (46) darstellt.

9. Vorrichtung nach Anspruch 8,
**dadurch gekennzeichnet**,
daß die Klemmscheibe (42) eine zu dem Vorsprung (36) korrespondierende und diesen aufnehmende Ausnehmung (48) aufweist.

10. Vorrichtung nach Anspruch 8 oder 9,
**dadurch gekennzeichnet**,
daß die Klemmscheibe (42) an ihrer dem Schraubenkopf (32) zugewandten Seite (44) mit wenigstens zwei einander diametral gegenüberliegenden, radial verlaufenden Kerben (50), Nuten zur teilweisen Aufnahme des den Vorsprung (36) durchgreifenden Spickdrahtes (24) versehen ist.

11. Vorrichtung nach Anspruch 6 bis 10,
**dadurch gekennzeichnet**,
daß die Klemmschrauben (26) jeweils mit einer Mutter (28) konterbar sind, die eine am Rahmenelement (12) zur Anlage kommende, verkippbare Scheibe (52) umfaßt.

12. Vorrichtung nach wenigstens einem der Ansprüche 1 bis 11,
**dadurch gekennzeichnet**,
daß die teleskopartig verlängerbaren, die Rahmenelemente (12) miteinander verbindenden und relativ zueinander festlegenden Spannelemente (16) mindestens zwei, vorzugsweise drei Gewindeabschnitte (54, 56, 58) umfassen, die wenigstens teilweise ineinander greifen.

13. Vorrichtung nach Anspruch 12,
**dadurch gekennzeichnet**,
daß ein erster Gewindeabschnitt (54) von einer zentralen Gewindespindel (60) und einer zentralen, mit der Gewindespindel (60) zusammenwirkenden Gewindehülse (62) gebildet ist, die jeweils endseitig, voneinander abgewandt einen zweiten und dritten Gewindeabschnitt (56, 58) einschraubbar aufnehmen.

14. Vorrichtung nach Anspruch 13,
**dadurch gekennzeichnet**,
daß der erste Gewindeabschnitt (54) zu dem zweiten und dritten Gewindeabschnitt (56, 58) entgegengesetzt drehend ausgebildet ist.

15. Vorrichtung nach Anspruch 14,
**dadurch gekennzeichnet**,
daß der erste Gewindeabschnitt (54) linksdrehend und der zweite sowie dritte Gewindeabschnitt (56, 58) rechtsdrehend ausgebildet sind.

16. Vorrichtung nach Anspruch 13 bis 15,
**dadurch gekennzeichnet**,
daß der erste Gewindeabschnitt (54) mit einer Markierung (64) zur genau vorherbestimmbaren Verlängerung bzw. Verkürzung des jeweiligen Spannelements (16) ausgestattet ist.

17. Vorrichtung nach Anspruch 16,
**dadurch gekennzeichnet**,
daß die zentrale Gewindespindel (60) in Mittellängsrichtung mit einer Nut (66), Kerbe versehen ist und die Gewindehülse (62) im Bereich ihrer Öffnung eine umfangsmäßig angeordnete Skalierung (68) aufweist.

18. Vorrichtung nach Anspruch 13 bis 17,
**dadurch gekennzeichnet**,
daß der zweite und dritte Gewindeabschnitt (56, 58) jeweils aus Gewindestangen (70) ausgebildet sind.

19. Vorrichtung nach Anspruch 18,
**dadurch gekennzeichnet**,
daß die beiden Enden (72, 74) einer jeden Gewindestange (70) koaxial zu der Mittellängsachse der Gewindestange (70) verlaufen.

20. Vorrichtung nach Anspruch 18,
**dadurch gekennzeichnet**,
daß die beiden Enden (72, 74) einer jeden Gewindestange (70) zueinander parallel verlaufen.

21. Vorrichtung nach Anspruch 20,
**dadurch gekennzeichnet**,
daß jede der Gewindestangen (70) einen mittleren Bereich (76) umfaßt, der gegenüber den beiden Enden (72, 74) einer jeden Gewindestange (70) bis zu einem Winkel von 90° abgewinkelt ausgebildet ist.

22. Vorrichtung nach Anspruch 18,
**dadurch gekennzeichnet**,
daß die beiden Enden (72, 74) einer jeden Gewindestange (70) zueinander bis zu einem Winkel von 180° abwinkelbar sind.

23. Vorrichtung nach Anspruch 22,
**dadurch gekennzeichnet**,
daß jede der Gewindestangen (70) einen mittleren Bereich (78) umfaßt, in dem ein scharnierartiges Gelenk (80) angeordnet ist.

24. Vorrichtung nach wenigstens einem der Ansprüche 12 bis 23,
**dadurch gekennzeichnet**,
daß der zweite und dritte Gewindeabschnitt (56, 58) schräg zu dem jeweiligen Rahmenelement (12) verlaufend an dem Rahmenelement (12) befestigbar sind.

25. Vorrichtung nach Anspruch 24,
**dadurch gekennzeichnet**,
daß der zweite und dritte Gewindeabschnitt (56, 58) über eine Mutter (92, 92') mit dem Rahmenelement (12) zugewandter Kalottenform und eine Zwischenscheibe (94, 94') mit zur Kalottenform der Mutter (92, 92') entsprechender, dem Rahmenelement (12) abgewandter kalottenförmiger Auflage (98, 98') an dem jeweiligen Rahmenelement (12) befestigbar sind.

26. Vorrichtung nach wenigstens einem der Ansprüche 6 bis 25,
**dadurch gekennzeichnet**,
daß die Rahmenelemente (12) und/oder die Spannelemente (16) und/oder die Klemmschrauben (26) aus Aluminium, insbesondere Duraluminium, bestehen.

## Claims

1. Device for lengthening bones, comprising at least two frame members (12) spaced apart and at least partly surrounding the bone, for receiving and fastening members (14) passing through the bone approximately perpendicularly to its longitudinal direction, and telescopically extensible clamp members (16) connecting the frame members together and fixing them relative to one another, the frame members (12) being respectively of a U-shaped or semi-oval design and having longitudinal slots (20,20',20'') for fixing the fastening members (14) and clamping members (16),
characterised in that
the longitudinal slots (20,20',20'') are disposed in two or more rows.

2. Device according to claim 1,
characterised in that
the longitudinal slots (20,20',20'') of two respectively adjacent rows (18,18') of longitudinal slots (20,20',20'') are disposed to overlap one another.

3. Device according to claim 1 and/or 2,
characterised in that
the rows (18,18') of longitudinal slots (20,20',20'') are interrupted by at least one transverse slot (22).

4. Device according to at least one of claims 1 to 3,
characterised in that
at least one longitudinal slot (20') of one or a plurality of the rows (18 or 18') of longitudinal slots (20,20',20'') is designed to extend towards an adjacent row (18 or 18') of longitudinal slots (20,20',20'').

5. Device according to at least one of claims 1 to 4,
characterised in that
at least one longitudinal slot (20'') of one or more of the rows (18,18') of longitudinal slots (20,20',20'') is freely accessible at the end of the U-shaped semi-oval frame member (12).

6. Device according to at least one of claims 1 to 5,
characterised in that
clamp screws (26) are provided, which are fixable to the frame members (12), for receiving and fixing the fastening members (14), the said fastening members (14) being in the form of spit wires (24).

7. Device according to claim 6,
characterised in that
the clamp screws (26) are respectively so designed that the clamp screws (26) for the spit wires (24) come into contact over a large area and thus in a rotationally stable manner on the frame member (12).

8. Device according to claim 6 or 7,
characterised in that
the clamp screws (26) are of a two-part design, one part (30) comprising a screw (32) and a helical thread (34), between which is located a projection (36) with a bore, preferably a central bore (38) for receiving the spit wire (24) with a diameter which is smaller than the diameter of the screw head (32) and larger than the diameter of the helical thread (34), and the other part (40) representing a clamp disc (42) with a side (44) facing the screw head (32) and acting on the spit wire, and with a side (46) facing away from the screw head (32) and abutting with its surface on the frame member (12).

9. Device according to claim 8,
characterised in that
the clamp screw (42) has a recess (48) corresponding to the projection (36) and receiving it.

10. Device according to claim 8 or 9,
characterised in that
the clamp disc (42) is provided on its side (44) facing the screw head (32) with at least two notches (50) or grooves, facing one another diametrically and extending radially, for partly accommodating the spit wire (24) passing through the projection (36).

11. Device according to claims 6 to 10,
characterised in that
the clamp screws 926) are respectively lockable by means of a nut (28), which comprises a tiltable disc (52) coming into contact with the frame member (12).

12. Device according to at least one of claims 1 to 11,
characterised in that
the telescopically extensible clamp members (16) connecting the frame members (12) together and fixing them relative to one another comprise at least two, preferably three threaded sections (54,56,58) which engage at least partly in one another.

13. Device according to claim 12,
characterised in that
a first threaded section (54) is formed by a central threaded rod (60) and a central threaded sleeve (62) cooperating with the threaded rod (60) and which, at the end, and facing away from one another, receive a second and third threaded section (56, 58) in a screw connection.

14. Device according to claim 13,
characterised in that
the first threaded section (54) is designed to rotate in a contrary direction to the second and third threaded sections (56, 58).

15. Device according to claim 14,
characterised in that
the first threaded section (54) is on a left-hand thread and the second and third threaded sections (56,58) are on a right-hand thread.

16. Device according to claims 13 to 15,
characterised in that
the first threaded section (54) is provided with a marking (64) for precisely predeterminable lengthening or shortening of the respective clamping member (16).

17. Device according to claim 16,
characterised in that
the central threaded rod (60) is provided in the central longitudinal direction with a groove (66) or notch, and the threaded sleeve (62) has in the area of its opening a scale (68) located on the periphery.

18. Device according to claims 13 to 17,
characterised in that
the second and third threaded sections (56,58) are respectively formed from threaded rods (70).

19. Device according to claim 18,
characterised in that
both ends (72,74) of each threaded rod (70) extend coaxially to the central longitudinal axis of the threaded rod (70).

20. Device according to claim 18,
characterised in that
both ends (72,74) of each threaded rod (70) extend parallel to one another.

21. Device according to claim 20,
characterised in that
each of the threaded rods (70) comprises a central area (76), which is angled relative to both ends (72,74) of each threaded rod (70) up to an angle of 90°.

22. Device according to claim 18,
characterised in that
both ends (72,74) of each threaded rod (70) may be angled relative to one another up to an angle of 180°.

23. Device according to claim 22,
characterised in that
each of the threaded rods (70) comprises a central area (78), in which there is located a hinge-like joint (80).

24. Device according to at least one of claims 12 to 23,
characterised in that
the second and third threaded sections (65,68) may be secured to extend obliquely to the respective frame member (12) on the frame member (12).

25. Device according to claim 24,
characterised in that
the second and third threaded sections (56,58) may be attached on the respective frame member (12) via a nut (92,92') with a domed shape facing the frame member (12) and an intermediate disc (94,94') with a dome-shaped contact surface (98,98') corresponding to the domed shape of the nut (92,92') and facing away from the frame member (12) on the respective frame member (12).

26. Device according to at least one of claims 6 to 25,
characterised in that
the frame members (12) and/or the clamping members (16) and/or the clamp screws (26) consist of aluminium, particularly duraluminium.

## Revendications

1. Dispositif pour prolonger des os, comportant au moins deux éléments de cadre (12) distants l'un de l'autre et enveloppant l'os, en partie du moins, pour recevoir et fixer des éléments de fixation (14) qui passent au travers de l'os à peu près à la perpendiculaire du sens longitudinal de ce dernier, ainsi que des éléments de serrage (16) qui peuvent être rallongés à la manière d'un télescope, assemblent les éléments de cadre entre eux, et les fixent mutuellement, les éléments de cadre (12) étant respectivement réalisés en forme de U ou de semi-ovale, et présentant des fentes longitudinales (20, 20', 20'') pour fixer les éléments de fixation (14) et les éléments de serrage (16), caractérisé en ce que les fentes longitudinales (20, 20', 20'') sont disposées en deux ou en plusieurs rangées.

2. Dispositif suivant la revendication 1, caractérisé en ce que les fentes longitudinales (20, 20', 20'') de deux rangées respectivement adjacentes (18, 18') de fentes (20, 20', 20'') se chevauchent mutuellement.

3. Dispositif suivant l'une des revendications 1 et 2, caractérisé en ce que les rangées (18, 18') de fentes longitudinales (20, 20', 20'') sont interrompues par une fente transversale (22), au moins.

4. Dispositif suivant l'une au moins des revendications 1 à 3, caractérisé en ce qu'au moins une fente longitudinale (20') d'une ou de plusieurs des rangées (18 et/ou 18') de fentes longitudinales (20, 20', 20'') est réalisée en direction d'une rangée adjacente (18' et/ou 18) de fentes longitudinales (20, 20', 20'').

5. Dispositif suivant l'une au moins des revendications 1 à 4, caractérisé en ce qu'au moins une fente longitudinale (20'') d'une ou de plusieurs des rangées (18, 18') de fentes longitudinales (20, 20', 20'') est librement accessible côté frontal sur l'extrémité de l'élément de cadre (12) en forme de U, de semi-ovale.

6. Dispositif suivant l'une au moins des revendications 1 à 5, caractérisé en ce que des vis de serrage (26), qui peuvent être fixées sur les éléments de cadre (12), sont prévues pour recevoir et fixer les éléments de fixation (14), les éléments de fixation (14) étant réalisés sous forme de fils de traverse (24).

7. Dispositif suivant la revendication 6, caractérisé en ce que les vis de serrage (26) ont respectivement une réalisation telle que les vis (26), prévues pour les fils de traverse(24), entrent au contact de l'élément de cadre (12) sur une grande portée, et sont donc stables en rotation.

8. Dispositif suivant l'une des revendications 6 et 7, caractérisé en ce que les vis de serrage (26) sont réalisées en deux parties, l'une des parties (30) comportant une tête de vis (32) et un filetage de vis (34), entre lesquels est disposé un épaulement (36) muni d'un trou, d'un trou central (38) de préférence, pour recevoir le fil de traverse(24), le diamètre de cet épaulement étant inférieur à celui de la tête de vis (32) et supérieur à celui du filetage de vis (34), et l'autre partie (40) constituant une rondelle de serrage (42) avec un côté (44) tourné vers la tête de vis (32) et sollicitant le fil de traverse, et avec un côté (46) opposé à la tête de vis (32) et s'appliquant d'une manière plane sur l'élément de cadre (12).

9. Dispositif suivant la revendication 8, caractérisé en ce que la rondelle de serrage (42) présente un évidement (48) correspondant à l'épaulement (36) et recevant ce dernier.

10. Dispositif suivant l'une des revendications 8 et 9, caractérisé en ce que la rondelle de serrage (42) est munie, sur son côté (44) tourné vers la tête de vis (42), d'au moins deux entailles (50), rainures, situées dans le sens radial et diamétralement en vis-à-vis, pour la réception partielle du fil traverse (24) passant au travers de l'épaulement (36).

11. Dispositif suivant les revendications 6 à 10, caractérisé en ce que les vis de serrage (26) peuvent être respectivement bloquées par un écrou (28), qui comporte une rondelle culbutable (52) entrant au contact de l'élément de cadre (12).

12. Dispositif suivant l'une au moins des revendications 1 à 11, caractérisé en ce que les éléments de serrage (16) qui peuvent être rallongés à la manière d'un télescope, assemblent entre eux les éléments de cadre (12) et les fixent mutuellement, comportent au moins deux sections filetées, trois de préférence (54, 56, 58), qui s'engagent les unes dans les autres, partiellement au moins.

13. Dispositif suivant la revendication 12, caractérisé en ce qu'une première section filetée (54) est formée par une broche filetée centrale (60) et par une douille taraudée centrale (62), concourant avec la broche filetée (60), dans lesquelles peuvent être respectivement vissées côté extrême une deuxième et une troisième section filetée (56, 58) opposées l'une à l'autre.

14. Dispositif suivant la revendication 13, caractérisé en ce que la première section filetée (54) est réalisée en rotation en sens contraire de la deuxième et de la troisième section filetée (56, 58).

15. Dispositif suivant la revendication 14, caractérisé en ce que la première section filetée (54) est réalisée en rotation à gauche, la deuxième et la troisième section filetée (56, 58) étant réalisées en rotation à droite.

16. Dispositif suivant les revendications 13 à 15, caractérisé en ce que la première section filetée (54) est munie d'un repère (64) pour le rallongement et/ou le raccourcissement exactement prédéfinissables de l'élément de serrage respectif (16).

17. Dispositif suivant la revendication 16, caractérisé en ce que la broche filetée centrale (60) est munie d'une rainure (66), entaille, dans le sens longitudinal central, la douille taraudée (62) présentant, dans la zone de son ouverture, une graduation (68) disposée sur son pourtour.

18. Dispositif suivant les revendications 13 à 17, caractérisé en ce que la deuxième et la troisième section filetée (56, 58) sont respectivement formées de tiges filetées (70).

19. Dispositif suivant la revendication 18, caractérisé en ce que les deux extrémités (72, 74) de chaque tige filetée (70) se situent dans le sens coaxial par rapport à l'axe longitudinal central de la tige filetée (70).

20. Dispositif suivant la revendication 18, caractérisé en ce que les deux extrémités (72, 74) de chaque tige filetée (70) sont parallèles entre elles.

21. Dispositif suivant la revendication 20, caractérisé en ce que chacune des tiges filetées (70) comporte une zone centrale (76), qui peut être coudée par rapport aux deux extrémités (72, 74) de chaque tige filetée (70) jusqu'à un angle de 90°.

22. Dispositif suivant la revendication 18, caractérisé en ce que les deux extrémités (72, 74) de chaque tige filetée (70) peuvent être coudées l'une par rapport à l'autre jusqu'à un angle de 180°.

23. Dispositif suivant la revendication 22, caractérisé en ce que chacune des tiges filetées (70) comporte une zone centrale (78), dans laquelle est disposée une articulation (80) en forme de charnière.

24. Dispositif suivant l'une au moins des revendications 12 à 23, caractérisé en ce que la deuxième et la troisième section filetée (56, 58) peuvent être fixées sur l'élément de cadre (12) en oblique de l'élément (12) respectif.

25. Dispositif suivant la revendication 24, caractérisé en ce que la deuxième et la troisième section filetée (56, 58) peuvent être fixées sur l'élément de cadre respectif (12) par un écrou (92, 92'), avec une forme de calotte tournée vers l'élément de cadre (12), et par une rondelle intermédiaire (94, 94') avec un appui (98, 98') en forme de calotte opposé à l'élément de cadre (12) et correspondant à la forme de calotte de l'écrou (92, 92').

26. Dispositif suivant l'une au moins des revendications 6 à 25, caractérisé en ce que les éléments de cadre (12) et/ou les éléments de serrage (16) et/ou les vis de serrage (26) se composent d'aluminium, de Duralumin en particulier.
